(19) Europäisches Patentamt / European Patent Office / Office européen des brevets

(11) **EP 3 081 232 A1**

(12) **EUROPEAN PATENT APPLICATION**
published in accordance with Art. 153(4) EPC

(43) Date of publication:
**19.10.2016 Bulletin 2016/42**

(51) Int Cl.:
**A61K 47/38** (2006.01)   **A61K 47/40** (2006.01)
**A61K 47/30** (2006.01)   **A61K 9/06** (2006.01)

(21) Application number: **14869008.4**

(22) Date of filing: **05.12.2014**

(86) International application number:
**PCT/KR2014/011912**

(87) International publication number:
**WO 2015/088198 (18.06.2015 Gazette 2015/24)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**

(30) Priority: **09.12.2013 KR 20130152585**

(71) Applicant: **New Medic Co., Ltd.**
**Asan-si, Chungcheongnam-do 336-795 (KR)**

(72) Inventors:
• **WOO, Hee Dong**
**Cheongju-si**
**Chungcheongbuk-do 363-782 (KR)**

• **HUR, Jung Mu**
**Asan-si**
**Chungcheongnam-do 336-728 (KR)**

(74) Representative: **dompatent von Kreisler Selting Werner -**
**Partnerschaft von Patent- und Rechtsanwälten mbB**
**Deichmannhaus am Dom**
**Bahnhofsvorplatz 1**
**50667 Köln (DE)**

(54) **HYALURONIC ACID GEL COMPOSITION HAVING SUSTAINED RELEASE PROPERTY**

(57)     Provided is a cross-linked polysaccharide gel composition used in cosmetics and medicine fields, and more particularly, to a hyaluronic acid gel composition having durability capable of maintaining durability for a long time within a living body without increasing a cross-linked bond rate by including a material having functions of decomposition inhibition and antioxidation of polysaccharide in cross-linked polysaccharide gel. In addition, the composition is a hydro gel complex in which ursolic acid is contained in non-cross-linked or cross-linked polysaccharide.

[Fig. 1]

Hyaluronic acid

Encapsulation of ursolic acid

EP 3 081 232 A1

## Description

[Technical Field]

The present invention relates to a cross-linked polysaccharide gel composition used in cosmetics and medicine fields, and more particularly, to a hyaluronic acid gel composition having durability capable of maintaining durability for a long time within a living body without increasing a cross-linked bond rate by including a material having functions of decomposition inhibition and antioxidation of polysaccharide in cross-linked polysaccharide gel.

[Background Art]

Polysaccharide is a complex of sugar in which monosaccharides are bonded through glycoside bond, and in particular, sodium hyaluronate is widely used in cosmetics and medicine fields.

Sodium hyaluronate is glycosaminoglycans present in the dermis layer, and a living body polymer material in which N-acetyl-D-glucosamine and D-glucuronic acid are connected by beta-1,3 glycosidic bond and the repeating units are linearly connected to each other.

In addition, sodium hyaluronate is a living body constituent that is found in a body fluid, eyeballs of a cow, a cockscomb, a buffer tissue of an animal, placenta, and so on, and in particular, present in a connective tissue and the skin at a high concentration.

In addition, since sodium hyaluronate can contain water of thousand times of its own weight, sodium hyaluronate is widely used as humectants of cosmetics of preventing dry of the skin. Further, in a skin care field, sodium hyaluronate is used as dermal filler or the like in wrinkle improvement, contour correction, or the like, as being inserted into a specific area to expand a soft tissue. Furthermore, since sodium hyaluronate has good viscoelasticity, biocompatibility and biodegradability, sodium hyaluronate is widely used in degenerative osteoarthristis medicine, scar medicine, eyeball surgery assistance, anti-agglutination agent for preventing agglutination between tissues after surgery, and so on, in addition to the skin care.

However, since natural hyaluronic acid (HA) is rapidly decomposed in a living body by hyaluronidase and free radicals and has a very short half-life period of about a day, in spite of excellence of such hyaluronic acid, the natural hyaluronic acid cannot be directly applied to a field that requires a long half-life period such as a wrinkle medicine field.

In order to overcome the above-mentioned disadvantages, various kinds of cross-linking technologies have been developed, many kinds of cross-linked hyaluronic acid fillers using these technologies are developed in Europe and US, and in recent years, several products have been released in Korea.

US Patent No. 4,582,865 discloses hyaluronic derivatives that are cross-linked using divinyl sulfone (DVS) as a cross-linking agent, and hydro gel thereof is released as a trade name of Hylaform. In addition, US Patent No. 5,827,937 discloses a method of manufacturing a hyaluronic acid derivative cross-linking material using multi-functional epoxy compounds as a cross-linking agent, and in the multi-functional epoxy compounds, restylene serving as hydro gel of a hyaluronic acid cross-linking material manufactured using 1,4-butanediol diglycidyl ether (BDDE) as a cross-linking agent is world-widely released as a tissue-enhancing filler under approval of FDA in US.

Such products are generated by bonding a hydroxy group of hyaluronic acid and a cross-linking agent. While durability of the products in the living body is increased in comparison with non-cross-linked hyaluronic acid, bio-durability is low, for example decomposition within 6 to 12 months, and so on.

Since most of the cross-linked hyaluronic acid fillers have durability of less than 1 year, attempts of increasing a cross-linking bond rate have been performed to increase the durability. However, since feeling of irritation is caused by high storage modulus, this method is restrictive.

Accordingly, in order to increase durability, instead of an increase in cross-linked bond rate, a polysaccharide gel composition capable of increasing durability while having a storage modulus similar to the skin should be developed.

[Summary of Invention]

[Technical Problem]

An object of the present invention is to provide a cross-linked hyaluronic acid gel composition having a long-term durability in a living body by including a material having functions of polysaccharide decomposition inhibition and antioxidation of cross-linked polysaccharide gel without increasing a cross-linking bond rate.

Another object of the present invention is to provide a cross-linked hyaluronic acid gel composition having a long-term durability, in a cross-linked polysaccharide gel composition used in cosmetics and medicine fields, including ursolic acid having functions of decomposition inhibition and antioxidation of polysaccharide in the cross-linked polysaccharide gel without an increase in cross-linked bond rate.

[Solution to Problem]

In order to achieve the aforementioned objects, the present invention is directed to a hydro gel complex including ursolic acid in non-cross-linked or cross-linked hyaluronic acid.

In addition, according to the present invention, the hydro gel composition includes at least one of polysaccharide selected from the group consisting of hyaluronic acid, cellulose, chitosan, dextran, dextran sulfate, chondroitin, chondroitin sulfate, heparin, heparin sulfate and alginate.

In addition, according to the present invention, a molecular weight of the polysaccharide is 20,000 Dalton (Da) to 5,000,000 Dalton (Da).

In addition, according to the present invention, a concentration of the ursolic acid is 0.001 mM to 1 M.

In addition, according to the present invention, lidocaine is further provided.

[Advantageous Effects of Invention]

According to the present invention, the cross-linked polysaccharide gel composition including the ursolic acid has good biocompatibility and provides excellent long-term durability in the living body while maintaining a storage modulus most similar to the skin when the composition is injected into the living body with no increase in storage modulus.

In addition, a composition into which lidocaine serving as a local anesthetic according to the present invention is added attenuates pains of a patient upon surgery.

In addition, the composition according to the present invention provides effects of removing or improving wrinkles of the skin and recovering the tissue in an area such as the nose, cheeks, lips, breasts, hips, a deep scar, or the like.

In addition, the composition according to the present invention is used as an agent for enlarging a man's symbol urologically and an anti-agglutination agent for preventing long-term agglutination after surgery, and used as a degenerative arthritis medicine serving as lubricant for improving and curing degenerative arthritis.

[Brief Description of Drawings]

Fig. 1 is a schematic view showing a complex structure of cross-linked hyaluronic acid and ursolic acid according to the present invention.
Fig. 2 is a graph showing a decomposition rate of a hyaluronic acid-ursolic acid complex by hyaluronidase and free radicals according to the present invention.
Fig. 3 is a graph showing rheology characteristics of the hyaluronic acid-ursolic acid complex according to the present invention.
Fig. 4 is a graph showing a squeeze force of the hyaluronic acid-ursolic acid complex according to the present invention.

[Description of Embodiment]

An embodiment of the present invention will be described in detail with reference to the accompanying drawings.

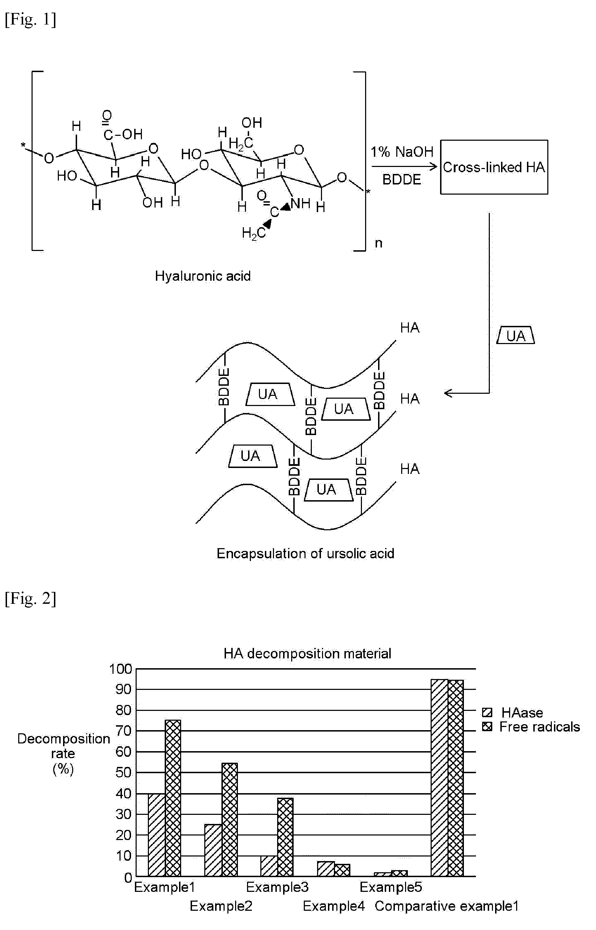

Fig. 1 is a schematic view showing a complex structure of cross-linked hyaluronic acid and ursolic acid according to the present invention.

As shown, a cross-linked polysaccharide gel composition having long-term durability according to the present invention is constituted by a hydro gel composition in which ursolic acid is included in non-cross-linked or cross-linked polysaccharide.

More specifically, the composition of the present invention is constituted by at least one polysaccharide selected from the group consisting of hyaluronic acid, cellulose, chitosan, dextran, dextran sulfate, chondroitin, chondroitin sulfate, heparin, heparin sulfate and alginate, and a hydro gel composition including ursolic acid serving as a material having functions of decomposition inhibition and antioxidation of the polysaccharide.

The polysaccharide uses at least one or more selected from the group consisting of hyaluronic acid, cellulose, chitosan, dextran, dextran sulfate, chondroitin, chondroitin sulfate, heparin, heparin sulfate and alginate, preferably, hyaluronic acid.

A molecular weight of the polysaccharide used in the present invention is 20,000 Dalton to 5,000,000 Dalton, preferably, 500,000 Dalton to 3,000,000 Dalton.

The ursolic acid used in the present invention is a pentacyclic triterpenoid-based compound known as urson, prunol, micromerol, malol, and so on, which is widely distributed in medicinal plant, herb, or the like.

The ursolic acid is considered to have pharmacological inactivity for a long time, and the ursolic acid and the sodium ursolate (e.g. potassium or sodium ursolates) have been used as a surfactant in medicine, cosmetics and foods.

However, it has been found that both of the ursolic acid and the sodium ursolate have pharmacological activity when the ursolic acid is locally taken through more precise research.

The ursolic acid exhibits effectiveness in anti-inflammatory action, anti-cancer effect (skin cancer), and anti-bacterial effect. Similar to most of triterpenoids-based compound, the ursolic acid is a compound that can be easily found in plants, and an ingredient of various kinds of plants in which a phylogeny origin and a classification position are diversified.

The ursolic acid is separated from peels of fruits such as an apple, a pear, a cranberry, a plum, and so on. In addition, ursolic acid derivatives are plentiful in seaweeds.

The ursolic acid used in the present invention is an excellent material of increasing durability in a living body of the polysaccharide gel because the ursolic acid has two functions of a polysaccharide decomposition inhibition agent and an antioxidant through a function of antioxidation while suppressing decomposition of the polysaccharide.

A concentration of the ursolic acid used in the present invention is 0.01 mM to 1 M, preferably, 0.05 mM to 100mM, and more preferably, 0.1 mM to 10 mM.

In addition, according to the present invention, in order to reduce pains of a patient during surgery, lidocaine serving as local anesthetic may be added to the cross-linked polysaccharide gel composition including the ursolic acid.

The composition of the present invention may be used to remove or improve wrinkles of the skin clinically or cosmetically, or recover the tissue of the area such as the nose, cheeks, lips, breasts, hips, deep scars, or the like.

In addition, the composition may be used as an anti-agglutination agent used to enlarge a man's symbol clinically or prevent long-term agglutination after surgery, or a degenerative arthritis medicine serving as lubricant for improvement and curing of degenerative arthritis.

Hereinafter, the present invention will be described in detail with reference to the following examples. However, the following examples are provided to exemplify the present invention but the scope of the present invention is not limited thereto.

Comparative Example 1: Preparation of cross-linked hyaluronic acid filler that was previously developed

100 uL of PBS (pH 7.0) solution was added to 1 g of cross-linked hyaluronic acid filler (REVOLAX$_{TM}$, Across Co., Ltd.) having a concentration of 24 mg/mL of HA and a storage modulus (G') or about 130 Pa, and then, agitated to be completely mixed.

Example 1: Manufacture of complex hydro gel of hyaluronic acid-ursolic acid (0.1mM) of invention

100 uL of PBS (pH 7.0) solution into which 0.1 mM of ursolic acid was added was added to 1 g of cross-linked hyaluronic acid filler (REVOLAX$_{TM}$, Across Co., Ltd) having a concentration of 24 mg/mL of HA and a storage modulus (G') of about 130 Pa, and then, agitated to be completely mixed.

Example 2: Manufacture of complex hydro gel of hyaluronic acid-ursolic acid (0.5mM) of invention

100 uL of PBS (pH 7.0) solution into which 0.5 mM of ursolic acid was added was added to 1 g of cross-linked hyaluronic acid filler (REVOLAX$_{TM}$, Across Co., Ltd) having a concentration of 24 mg/mL of HA and a storage modulus (G') of about 130 Pa, and then, agitated to be completely mixed.

Example 3: Manufacture of complex hydro gel of hyaluronic acid-ursolic acid (1mM) of invention

100 uL of PBS (pH 7.0) solution into which 1mM of ursolic acid was added was added to 1 g of cross-linked hyaluronic acid filler (REVOLAX$_{TM}$, Across Co., Ltd) having a concentration of 24 mg/mL of HA and a storage modulus (G') of about 130 Pa, and then, agitated to be completely mixed.

Example 4: Manufacture of complex hydro gel of hyaluronic acid-ursolic acid (5mM) of invention

100 uL of PBS (pH 7.0) solution into which 5 mM of ursolic acid was added was added to 1g of cross-linked hyaluronic acid filler (REVOLAX$_{TM}$, Across Co., Ltd) having a concentration of 24 mg/mL of HA and a storage modulus (G') of about 130 Pa, and then, agitated to be completely mixed.

Example 5: Manufacture of complex hydro gel of hyaluronic acid-ursolic acid (10mM) of invention

100 uL of PBS (pH 7.0) solution into which 10 mM of ursolic acid was added was added to 1 g of cross-linked hyaluronic acid filler (REVOLAX$_{TM}$, Across Co., Ltd) having a concentration of 24 mg/mL of HA and a storage modulus (G') of about 130 Pa, and then, agitated to be completely mixed.

Experiment Example 1: Decomposition test of hyaluronidase

In order to expect durability in a living body of the hyaluronic acid-ursolic acid complex according to the present invention, decomposition tests by a hyaluronic acid decomposition enzyme of the hyaluronic acid-ursolic acid complex of the comparative example 1 and the examples 1 to 5 have been performed.

The hyaluronic acid hydro gels of the comparative example 1 and the examples 1 to 5 were frozen and dried, and the frozen and dried samples of the same mass were put into 50 mL tubes. 6mL of PBS solution (pH 7.0) including 200 units of hyaluronic acid decomposition enzyme (hyaluronidase from Streptomyces hyalurolyticus, Sigma-Aldrich) was added. The mixture was heated at 90°C for 10 minutes to inactivate the hyaluronic acid decomposition enzyme after reaction at 37°C for 24 hours. 18mL of purified water was added and agitated, and then, centrifugally separated (4,000 rpm for 15 minutes). 1mL of supernatant was put into an epp. tube and centrifugally separated (12,000 rpm for 5 minutes). An amount of N-acetylglucosamine (NAG) decomposed and emitted from the centrifugally separated supernatant was measured through analysis according to a carbazole method. Decomposition levels of the cross-linked material are shown in the following Table 1.

[Table 1]

|  | Ursolic acid contents (mM) | Decomposition rate (%) | Relative decomposition rate (%) |
|---|---|---|---|
| Example 1 | 0.1 mM | 40.2 | 42.3 |
| Example 2 | 0.5 mM | 25.3 | 26.6 |
| Example 3 | 1 mM | 10.4 | 10.9 |
| Example 4 | 5 mM | 7.2 | 7.6 |
| Example 6 | 10 mM | 2.1 | 2.2 |
| Comparative Example 1 | 0 mM | 95.0 | 100.0 |

As shown in Table 1, it has been found that decomposition of the cross-linked hyaluronic acid is affected by a concentration of the ursolic acid. It has been observed that decomposition of the cross-linked hyaluronic acid by the hyaluronic acid decomposition enzyme (hyaluronidase) is remarkably decreased as the concentration of the ursolic acid is increased.

In addition, when the concentration of the ursolic acid is 10mM, little decomposition of the cross-linked hyaluronic acid by the hyaluronic acid decomposition enzyme (hyaluronidase) occurs. When the concentration of the ursolic acid is adjusted based on this, the hyaluronic acid hydro gel filler having desired durability can be manufactured.

Experiment Example 2: Free radicals decomposition teat

In order to expect durability in the living body of the hyaluronic acid-ursolic acid complex according to the present invention, decomposition tests by free radicals of the hyaluronic acid-ursolic acid complex of the comparative example 1 and the examples 1 to 5 were performed.

The hyaluronic acid hydro gels of the comparative example 1 and the examples 1 to 5 are frozen and dried, and the frozen and dried samples of the same amount were put into 50mL tubes.

6mL of PBS solution (pH 7.0) including free radicals (a mixture of 0.2 mM of ascorbic acid and 0.2 mM of hydrogen peroxide) was added. 18mL of purified water was added and agitated, and then, centrifugally separated (4,000 rpm for 15 minutes). 1ml of a supernatant was put into the epp. tube to be centrifugally separated (12,000 rpm for 5 minutes).

An amount of N-acetylglucosamine (NAG) decomposed and emitted from the centrifugally separated supernatant was measured through analysis according to a carbazole method. Decomposition levels of the cross-linked material are shown in the following Table 2.

[Table 2]

|  | Ursolic acid contents (mM) | Decomposition rate (%) | Relative decomposition rate (%) |
|---|---|---|---|
| Example 1 | 0.1mM | 75.8 | 80.0 |
| Example 2 | 0.5mM | 54.5 | 57.6 |

(continued)

|  | Ursolic acid contents (mM) | Decomposition rate (%) | Relative decomposition rate (%) |
|---|---|---|---|
| Example 3 | 1MAM | 38.0 | 40.1 |
| Example 4 | 5mM | 5.9 | 6.2 |
| Example 6 | 10mM | 3.1 | 3.3 |
| Comparative Example 1 | 0mM | 94.7 | 100.0 |

As shown in Table 2 and Fig. 2, it has been found that decomposition of the cross-linked hyaluronic acid is affected according to the concentration of the ursolic acid. It has been observed that decomposition of the cross-linked hyaluronic acid by the free rascals was remarkably decreased as the concentration of the ursolic acid is increased.

In addition, when the concentration of the ursolic acid is 10mM, little decomposition of the cross-linked hyaluronic acid by the free radicals occurs. When the concentration of the ursolic acid is adjusted based on this, the hyaluronic acid hydro gel filler having desired durability can be manufactured.

Experiment Example 3: Rheology characteristics test

In order to estimate rheology characteristics of the present invention, a storage modulus (G'), a loss modulus (G") and a degree of elasticity (%) of the hyaluronic acid-ursolic acid complex of the comparative example 1 and the examples 1 to 5 were measured using Rheometer (T.A. Instruments Ltd., USA). The test was performed within a range of 0.01 to 10 Hz using a 40 mm 2° cone-plate geometer at 25 °C, and the storage modulus (G') and the loss modulus (G") were measured at 1.0Hz. The degree of elasticity (%) was measured using the following equation, and resultant values are shown in the following table 3.

$$\text{Percentage elasticity (\%)} = (G'/G'+G'') \times 100$$

[Table 3]

|  | Storage modulus (G') | Loss modulus (G") | Degree of elasticity (%) |
|---|---|---|---|
| Example 1 | 132.46 | 31.31 | 80.88 |
| Example 2 | 122.53 | 29.48 | 80.61 |
| Example 3 | 135.80 | 30.07 | 81.87 |
| Example 4 | 134.75 | 31.24 | 81.18 |
| Example 6 | 126.89 | 30.29 | 80.73 |
| Comparative Example 1 | 129.26 | 30.29 | 81.02 |

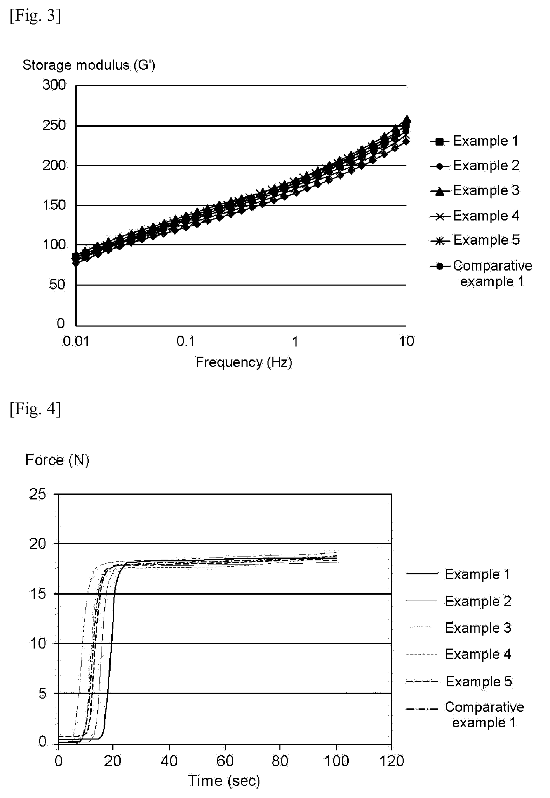

As shown in Table 3 and Fig. 3, addition of the ursolic acid did not vary the rheology characteristics. The cross-linked hyaluronic acid filler to which the ursolic acid is not added and the cross-linked hyaluronic acid filler to which the ursolic acid is added to a concentration of 0.1 to 10 mM hardly affected the storage modulus (G'), loss modulus (G") and the degree of elasticity (%). When the concentration of the ursolic acid is adjusted based on this, the hyaluronic acid hydro gel filler capable of increasing durability can be manufactured without increasing the storage modulus (G').

Experiment Example 4: Squeeze force test

In order to estimate the squeeze force of the present invention, squeeze force tests of the hyaluronic acid-ursolic acid complex of the comparative example 1 and the examples 1 to 5 were performed using a squeeze force tester.

A glass syringe in which test liquid to be measured was put was inserted into a jig, and then, a push rod of the glass syringe was adjusted to be disposed at a center of a pressing plate. A tray (a Petri dish) was installed such that the test liquid is stuck to the jig, and then, squeeze force measurement was performed at a speed of 12 mm/min.

From the measured result, a point of 5 mm after application of a force was designated as a bottom marker position and a point of 5 mm forward from the measurement-terminated point was designated as a top marker position, and then, an average value therebetween was taken. The squeeze force values are shown in the following table 4.

[Table 4]

|  | Maximum squeeze force (N) | Minimum squeeze force (N) | Average squeeze force (N) |
|---|---|---|---|
| Example 1 | 18.5 | 18.3 | 18.4 |
| Example 2 | 18.3 | 17.9 | 18.0 |
| Example 3 | 19.2 | 18.3 | 18.8 |
| Example 4 | 18.5 | 17.5 | 17.9 |
| Example 6 | 18.4 | 18.0 | 18.1 |
| Comparative Example 1 | 18.8 | 18.1 | 18.4 |

As shown in Table 4 and Fig. 4, addition of the ursolic acid did not vary the squeeze forces. The cross-linked hyaluronic acid filler to which the ursolic acid is not added and the cross-linked hyaluronic acid filler to which the ursolic acid is added to a concentration of 0.1 to 10 mM hardly affected the squeeze forces. When the concentration of the ursolic acid is adjusted based on this, the hyaluronic acid hydro gel filler capable of increasing durability can be manufactured while maintaining appropriate squeeze forces.

**Claims**

1.  A hyaluronic acid gel composition having durability, the composition comprising: ursolic acid contained in non-cross-linked or cross-linked hyaluronic acid.

2.  The hyaluronic acid gel composition having durability according to claim 1, wherein a molecular weight of the hyaluronic acid is 20,000 Dalton (Da) to 5,000,000 Dalton (Da).

3.  The hyaluronic acid gel composition having durability according to claim 1, wherein a concentration of the ursolic acid is 0.001 mM to 1 M.

4.  The hyaluronic acid gel composition according to claim 1, further comprising: lidocaine.

[Fig. 1]

Hyaluronic acid

Encapsulation of ursolic acid

[Fig. 2]

[Fig. 3]

Storage modulus (G')

[Fig. 4]

Force (N)

## INTERNATIONAL SEARCH REPORT

International application No.

**PCT/KR2014/011912**

| A. CLASSIFICATION OF SUBJECT MATTER |
|---|
| *A61K 47/38(2006.01)i, A61K 47/40(2006.01)i, A61K 47/30(2006.01)i, A61K 9/06(2006.01)i* |
| According to International Patent Classification (IPC) or to both national classification and IPC |

| B. FIELDS SEARCHED |
|---|
| Minimum documentation searched (classification system followed by classification symbols) |
| A61K 47/38; A61K 9/113; A61K 31/728; A61K 47/32; A61K 47/30; A61K 31/047; A61K 47/40; A61K 9/06 |
| Documentation searched other than minimum documentation to the extent that such documents are included in the fields searched<br>Korean Utility models and applications for Utility models: IPC as above<br>Japanese Utility models and applications for Utility models: IPC as above |
| Electronic data base consulted during the international search (name of data base and, where practicable, search terms used)<br>eKOMPASS (KIPO internal) & Keywords: hyaluronic acid, ursolic acid, gel composition, durability |

| C. DOCUMENTS CONSIDERED TO BE RELEVANT | | |
|---|---|---|
| Category* | Citation of document, with indication, where appropriate, of the relevant passages | Relevant to claim No. |
| A | KR 10-2012-0038662 A (AMOREPACIFIC CORPORATION) 24 April 2012<br>See claims 1-12. | 1-4 |
| A | KR 10-2011-0084510 A (ANTEIS S.A.) 25 July 2011<br>See claim 1. | 1-4 |
| A | JP 4822092 B2 (TAISHO PHARMACEUT CO LTD) 24 November 2011<br>See claims 1-7. | 1-4 |

| ☐ Further documents are listed in the continuation of Box C. | ☒ See patent family annex. |
|---|---|

| * Special categories of cited documents: | "T" later document published after the international filing date or priority date and not in conflict with the application but cited to understand the principle or theory underlying the invention |
|---|---|
| "A" document defining the general state of the art which is not considered to be of particular relevance | |
| "E" earlier application or patent but published on or after the international filing date | "X" document of particular relevance; the claimed invention cannot be considered novel or cannot be considered to involve an inventive step when the document is taken alone |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search | Date of mailing of the international search report |
|---|---|
| 10 FEBRUARY 2015 (10.02.2015) | **02 MARCH 2015 (02.03.2015)** |

| Name and mailing address of the ISA/KR | Authorized officer |
|---|---|
| Korean Intellectual Property Office<br>Government Complex-Daejeon, 189 Seonsa-ro, Daejeon 302-701,<br>Republic of Korea | |
| Facsimile No. 82-42-472-7140 | Telephone No. |

Form PCT/ISA/210 (second sheet) (July 2009)

**INTERNATIONAL SEARCH REPORT**
Information on patent family members

International application No.

**PCT/KR2014/011912**

| Patent document cited in search report | Publication date | Patent family member | Publication date |
|---|---|---|---|
| KR 10-2012-0038662 A | 24/04/2012 | CN 103167868 A | 19/06/2013 |
| | | KR 10-2012-0140525 A | 31/12/2012 |
| | | US 2013-0202667 A1 | 08/08/2013 |
| | | WO 2012-050359 A2 | 19/04/2012 |
| | | WO 2012-050359 A3 | 05/07/2012 |
| KR 10-2011-0084510 A | 25/07/2011 | CA 2742099 A1 | 14/05/2010 |
| | | CN 102196805 A | 21/09/2011 |
| | | CN 102196805 B | 16/10/2013 |
| | | EP 2349203 A2 | 03/08/2011 |
| | | EP 2349203 B1 | 30/10/2013 |
| | | EP 2676658 A1 | 25/12/2013 |
| | | JP 05-626661 B2 | 19/11/2014 |
| | | JP 2012-508217 A | 05/04/2012 |
| | | US 2011-0201571 A1 | 18/08/2011 |
| | | US 8455465 B2 | 04/06/2013 |
| | | WO 2010-052430 A2 | 14/05/2010 |
| | | WO 2010-052430 A3 | 07/10/2010 |
| JP 4822092 B2 | 24/11/2011 | JP 2005-097292 A | 14/04/2005 |

Form PCT/ISA/210 (patent family annex) (July 2009)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 4582865 A

- US 5827937 A